# EUROPÄISCHE PATENTSCHRIFT

(11) **EP 2 822 518 B1**
(45) Veröffentlichungstag und Bekanntmachung des Hinweises auf die Patenterteilung: **04.05.2016**
(21) Anmeldenummer: 13713737.8
(22) Anmeldetag: 07.03.2013
(51) Int. Cl.: A61F 7/10, A61N 7/00, A61B 90/00, A61F 7/02

(54) **GERÄT FÜR DIE KRYOLIPOLYSE**
DEVICE FOR CRYOLIPOLYSIS
APPAREIL DE CRYOLIPOLYSE

(30) Priorität: 08.03.2012 DE 202012002278 U
(43) Veröffentlichungstag der Anmeldung: 14.01.2015
(73) Patentinhaber: Lotsch, Friedemann, 44289 Dortmund (DE)
(72) Erfinder: Lotsch, Friedemann, 44289 Dortmund (DE)
(74) Vertreter: Vonnemann, Gerhard
(86) Internationale Anmeldenummer: PCT/EP2013/000678
(87) Internationale Veröffentlichungsnummer: WO 2013/131653

(56) Entgegenhaltungen:
- DE-A1- 4 320 443
- US-A1- 2003 220 674
- US-A1- 2008 077 211
- US-A1- 2010 280 582
- US-A1- 2011 313 411

## Beschreibung

Die Erfindung betrifft einen Applikator zur Verwendung in Verbindung mit einem Gerät zur Behandlung von subkutanen fettreichen Zellen mit Kälte.

Außerdem betrifft die Erfindung ein Gerät zur Behandlung von subkutanen fettreichen Zellen mit Kälte.

Bekanntermaßen werden zur physikalisch-medizinischen Behandlung bestimmter Symptome, beispielsweise in der Rheumatologie und der Sportmedizin, die betroffenen Körperteile gekühlt, wobei nach dem Stand der Technik verschiedene Methoden angewendet werden.

So ist es üblich, für die medizinische Kältebehandlung Gase aus Druckbehältern auf die Haut zu sprühen und dadurch schnelle und wirksame Kühlergebnisse zu erreichen. Dabei wird mit physiologisch bedenklichen Gasen, wie Propan, gearbeitet, die nicht eingeatmet werden dürfen, dermatologisch bedenklich und außerdem feuergefährlich sind.

Es ist ferner eine Vorrichtung zur partiellen Vereisung von Oberflächen, insbesondere zur Behandlung von oberflächigen Hautveränderungen bekannt, wobei das Handhabungsgerät mit Peltier-Modulen ausgestattet ist. Hierbei wird der aus dem Handhabungsgerät herausgeführte Behandlungskörper mit der kalten Fläche eines Peltier-Moduls wärmeleitend verbunden. Das Peltier-Modul selbst ist auf einer Wärmeverteilplatte gelagert und an einem Wärmeaustauscher angeschlossen, dessen Kühlkreislauf durch einen Leitungskreislauf mit der Energiegebereinheit und/oder einem Kühlwasseranschluß verbunden ist. Durch die gegebene Kombination mit

Einlagerung eines Peltier-Elementes ist das betreffende Handhabungsgerät entsprechend schwergewichtig und auch kompliziert im Aufbau. Darüber hinaus ist durch die Energieabstrahlung bzw. -übertragung über mehrere Austauschflächen ein erhöhter Energieaufwand erforderlich.

Eine Verbesserung ist bei einer bekannten Vorrichtung erzielt worden, bei der die Behandlungssonde selbst als Entspannungs- und/oder Durchflussraum für das Kältemittel ausgebildet ist. Dabei ist die Behandlungssonde als rohrförmiger Körper mit Entspannungs- und/oder Durchflussraum und einem vorderen Behandlungskörper ausgeführt worden. Durch die unmittelbare Kühlmittelzufuhr in den Durchflussraum bzw. die Entspannung des Kältemittels in dem Entspannungsraum der Behandlungssonde ist eine wesentlich bessere Kühlwirkung gegeben und damit eine weitaus geringere Energiezufuhr notwendig. Darüber hinaus wird duch Wegfall der sonst üblichen Peltier-Elemente ein besonders handliches und leichtes Gerät geschaffen.

Überdies ist eine sehr exakte Temperaturbestimmung und sofortige Reaktion der Behandlungssonde sichergestellt.

Ferner ist eine Behandlungssonde bekannt, die aus einem mit einem Behandlungskopf verbundenen und/oder austauschbar verbindbaren Energieteil besteht und der Behandlungskopf aus einer elastischen Oberfläche besteht.

Die bekannten Applikatoren sind meist sehr speziell an den jeweiligen medizinischen Anwendungsfall angepasst. Das therapeutische Ziel bestimmt das Temperaturniveau, die Behandlungsdauer und den Behandlungsort. Um diese Vorgaben zu erfüllen sind beispielsweise flexible Applikatoren aus der DE 296 11 440 U1 bekannt. Im Handel sind auch aus Kunststoff gefertigte Manschetten bekannt, die sich der Körperform anpassen.

Nachteilig an den flexiblen Manschetten ist der durch die Verwendung von Kunststoffen bedingte geringere Wärmeübergang. Feste Applikatoren schmiegen sich wiederum nicht der Körperform an, so dass sich deren Einsatz häufig schon deshalb verbietet. Werden sie jedoch mechanisch speziell so geschwächt, dass sie sich in gewissen Umfang an die Oberfläche eines Körpers anschmiegen können, wie in der DE 296 11 440 U1 vorgeschlagen, so führt dies zu einem erheblichen Fertigungsaufwand.

Für die Behandlung von subkutanen fettreichen Zellen wie sie beispielsweise in der WO 2010/127315 beschrieben wird, kommen kombinierte Konstruktionen zum Einsatz, da es auf eine genaue Temperaturführung ankommt. Die speziellen Anforderungen in diesem Bereich sind Gegenstand der WO 2007 133839 A1, WO 2008 039556 A1, WO 2009 011708 A1 und der US20030220674.

Aufgabe der Erfindung ist es, unter Beibehaltung einer optimalen Kühlwirkung einen wesentlich verbesserten Oberfiächenkontakt auch für großflächige Partien und Körperteile sicherzustellen und dabei eine möglichst günstige Wärmeabfuhr zu ermöglichen.
[A01] Bei einem Applikator zur Verwendung in Verbindung mit einem Gerät zur Behandlung von subkutanen fettreichen Zellen mit Kälte wird diese Aufgabe dadurch gelöst, dass der Applikators eine Kontaktfläche aufweist, die als eine Oberfläche einer festen Behandlungsplatte , vorzugsweise aus Aluminium, geformt ist. Durch die feste Platte werden die Einsatzbedingungen vorteilhaft reproduzierbar und der Prozess genauer steuerbar. Gleichzeitig wird durch Wahl eines besonders gut wärmeleitenden Materials die Körperwärme schneller dem Körper entzogen.
[A02] Damit die Behandlungsplatte eine gleich bleibende niedrige Temperatur beibehält, ist vorgesehen, dass in der festen Behandlungsplatte Kanäle zum Leiten eines Wärmeträgers vorgesehen sind. Als Wärmeträger kommt bevorzugt eine Sole zum Einsatz, deren Vorlauftemperatur genau geregelt ist und ca. 2° Celsius beträgt. Aus der Temperaturdifferenz zwischen Vor- und Rücklauf sowie dem Massenstrom ermittelt die Steuerung die abgeführte Wärmemenge. Die Kanäle können im Inneren der Platte vorgesehen sein oder als abgedeckte Gräben in der Oberfläche der Platte.
[A03] Dadurch, dass die feste Behandlungsplatte mit einer weiteren festen Platte zu einem Stapel geschichtet verbunden ist, erhöht sich die mechanische Festigkeit und ergibt eine gleichmäßigere Temperaturverteilung.
[A04] Besonders günstig lassen sich die Kanäle für den Wärmeträger zur Kühlung der Behandlungsplatte herstellen, wenn die Kanäle in einer Grenzfläche der beiden fest verbundenen Platten verlaufen. Die Kanäle werden dann in eine oder beide Platten als zunächst offene Nuten eingetieft. Nach dem Zusammenfügen der Platten entstehen dann geschlossene Kanäle. Diese können meanderförmig über die ganze Platte verteilt werden, um eine gleichmäßige Kühlung der gesamten Oberfläche zu erreichen.
   In die Kanäle können auch Rohre oder Schläuche eingebracht werden.
[A05] Die Maßnahme, dass eine äußere den Stapel abdeckende Isolierschicht vorgesehen ist, dient ebenfalls der verbesserten Kühlung. Die Schicht kann vorteilhaft aus einem verdichteten Polyurethanschaum bestehen, der parasitäre Wärmeströme in die Behandlungsplatte aus der Umgebung abschirmt, so dass auch die Ermittlung der aus dem Körper abgeführten Wärmemengen genauer wird. Dies lässt sich weiter verbessern, wenn zwischen Isolierschicht und weiterer fester Platte ein Spalt vorgesehen ist. Wenn der Spalt zusätzlich vakuumbeaufschlagt ausgebildet ist, wird durch die geringere Gasdichte im Spalt der Wärmeübergang noch verringert. Zusätzlich kann zwischen Kontaktfläche und Spalt vorteilhafterweise eine Verbindung vorgesehen sein, so dass durch das Vakuum auch die zu behandelnde Körperfläche an die Kontaktfläche angesogen wird. In dem Raum zwischen der Kontaktfläche und der zu behandelnden Fläche entsteht so ein Unterdruck, der zu einem sicheren Halt des Aplikators auf dem Körperteil führt. Die äußere Isolierschicht wird mit Vorteil durch entsprechende Formgestaltung zu einem Gehäuse geformt, so dass sie auch die Funktion eines Gerätegehäuses übernimmt.
[A06] Zur sicheren Auflage der Behandlungsplatte auf eine Körperstelle weist der Applikator mindestens eine in oder um die Kontaktfläche angeordnete unterdruckbeaufschlagte Öffnung auf. Dadurch saugt sich die Platte an die Körperhaut an, sobald Unterdruck an der Öffnung angelegt ist. Dies führt zu einer besonders gut reproduzierbaren Anlage der Oberfläche an die Haut.
[A07] Die Massnahme, dass die unterdruckbeaufschlagte Öffnung in einem die Kontaktfläche einrahmenden umlaufende Nut mündend angeordnet ist, dient ebenfalls der Verbesserung der Anlage der Behandlungsplatte. Die umlaufende Nut zieht die Luft unter der Kontaktfläche schneller ab.
[A03] Mit Vorteil ist die Nut in einem festen, die Schichten einfassenden Rahmen angeordnet. Zum Rahmen ist dann lediglich eine unterdruckbeaufschlagte Anschlussleitung notwendig und zur Behandlungsplatte Vor- und Rücklaufleitung für den Wärmeträger.
[A09] Zur sicheren Anlage der Behandlungsplatte ist weiter vorgesehen, dass eine die Kontaktfläche einrahmende umlaufende Dichtung vorgesehen ist, die vorzugsweise leicht austauschbar ist. Leckströme werden mit Vorteil verringert, so dass das Unterdruckaggregat in seiner Leistung geringer ausgelegt werden kann und unnötige Geräusche vermieden werden. Die Dichtung begrenzt nach außen den Raum zwischen Kontaktfläche und zu behandelnder Körperfläche, so dass der so gebildete Raum auch mit geringer Saugleistung mit Unterdruck beaufschlagt werden kann, da keine großen Leckströme entstehen. Da die Dichtung vorzugsweise abnehmbar ist, kann diese leicht gereinigt und ggf. desinfiziert werden. Ebenso wie die unter der Dichtung liegenden Anlageflächen.
[A10] Dadurch, dass die Dichtung in Richtung umschlossener Kontaktfläche diese teilweise überdeckend und keilförmig auslaufend geformt ist und im Übergangsbereich quer zum Verlauf der Dichtung gerichtete Kanäle vorgesehen sind, besteht die Möglichkeit auch stärker gewölbte Körperteile mit demselben Applikator zu behandeln. Durch die Formgebung wird eine zu große Kontaktfläche verringert. Für den Fall, dass Verbindungskanäle zum Unterdruckbereich in der Peripherie der Dichtung münden, kann im Übergangsbereich mindestens ein quer zum Verlauf der Dichtung gerichteter Kanal vorgesehen sein, der den Unterdruck in den Raum zwischen Kantaktfläche und zu behandelnder Körperfläche leitet. Die Dichtung schmiegt sich so an die Körperoberfläche an. Durch den Unterdruck kommt die Bandlungsfläche zur Anlage an die Kontaktfläche.
[A11] Die Behandlung lässt sich umfangreich dokumentieren, weil ein Temperatursensor vorgesehen ist, der vorzugsweise auf der Kontaktfläche oder in der Kühlplatte eingebaut angeordnet ist. Dieser Sensor liefert das Signal zur Steuerung, um bei Unterschreiten eines Schwellwertes beispielsweise die Kühlung abzuschalten und/oder um unmittelbar die Hauttemperatur zu dokumentieren.
[A12] In weiter Ausgestaltung ist ein Ultraschallschwinger in der Behandlungsplatte vorgesehen. Dadurch lässt sich das Gewebe zusätzlich stimulieren und die Wirkung des Applikators mit Vorteil noch verstärken. Wenn die Behandlungsplatte in mindestens einer Richtung konkav ausgebildet ist, wird die Anlage an verschiedene gekrümmte Körperflächen, insbesondere am Bauch, an der Hüfte, den Oberschenkeln etc. noch verbessert.
[A13] Bei der Behandlung von Oberschenkel-Innenseiten ist es erfindungsgemäß von Vorteil, wenn zwei Behandlungsplatten zu einem Stapel mit beidseitig angeordneten Kontaktflächen verbunden sind. Dann können beide inneren Schenkelinnenflächen gleichzeitig behandelt werden, indem der Applikator zwischen die Oberschenkel eingeklemmt wird.
[A14] Mit Vorteil kann auch der Applikator ein Gehäuse aufweisen, dessen Innenraum vakuumbeaufschlagt ausgebildet ist, und in das Versorgungsleitungen vakuumdicht einmünden. Das Gehäuse übernimmt auf diese Weise gleichzeitig die Funktion eines Unterdruckverteilers. Durch die geeignete Platzierung von Verbindungsleitungen, die in das Innere des Gehäuses führen, kann der Unterdruck auch in den an die Kontaktfläche angrenzenden Raum geleitet werden. Das Gewicht des Applikators ist seinen sicheren Halt auf der zu behandelnden Fläche nicht notwendig. Stattdessen erzeugt der Unterdruck die notwendige Haltekraft. Deshalb kann das Gewicht des Applikators durch einen Balancier kompensiert werden, so dass er leicht zu handhaben ist.
[A15] Die Aufgabe wird auch durch ein Gerät zur Behandlung von subkutanen fettreichen Zellen mit Kälte gelöst, das aus einem mit einem Wärmeträgerkreislauf und einem von einem Wärmeträger durchflossenen Applikator gemäß mindestens einem der vorstehenden Ansprüche besteht und mit einem Kältegerät, zur Bereitstellung des Wärmeträgers mit einer bestimmten Temperatur und mit verbindenden Versorgungsleitungen zum Applikator sowie einem das Gewicht des Applikators kompensierenden Balancier. Als Balancier ist in diesem Zusammenhang jede das Gewicht des Applikators kompensierende Vorrichtung.

Eine bevorzugte Ausführungsform der Erfindung wird beispielhaft an Hand einer Zeichnung erläutert. Die Figuren der Zeichnung zeigen im Einzelnen:
- Figur 1: einen schematischen Querschnitt durch einen nicht erfindungsgemäßen Applikator,
- Figur 2: einen schematischen Querschnitt durch eine Ausgestaltung des erfindungsgemäßen Applikators,
- Figur 3: eine Seitenansicht eines Beinapplikators,
- Figur 4: eine Aufsicht auf einen Beinapplikator gemäß Figur 3,
- Figur 5: einen Vertikalschnitt durch einen Beinapplikator gemäß Schnittlinie A-A in Figur 3,
- Figur 6: einen Vertikalschnitt durch einen Beinapplikator gemäß Schnittline VI-VI in Figur 5,
- Figur 7: einen Vertikalschnitt durch einen Beinapplikator gemäß Schnittline VII-VII in Figur 5 und
- Figur 8: eine perspektivische Ansicht des Geräts.

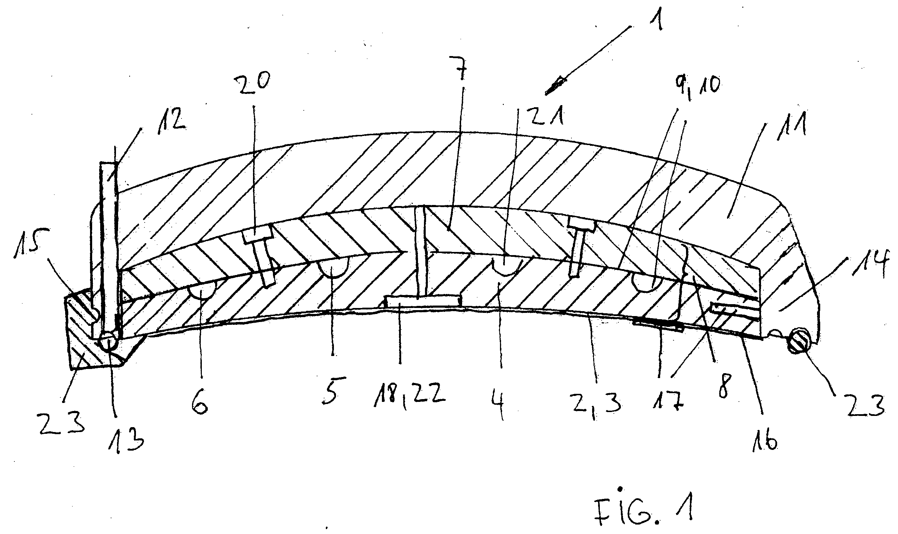

In Figur 1 ist der Querschnitt durch eine bevorzugte Ausführungsform des offenbarten Applikators 1 gezeigt. Dieser besteht aus einer festen Behandlungsplatte 4, deren Oberfläche 3 im Einsatz die Kontaktfläche 2 zu der Haut einer zu behandelnden Person herstellt. Damit die Körperwärme mit einem möglichst geringen Wärmeleitwiderstand abgeleitet werden kann, ist das bevorzugte Material dieser Behandlungsplatte 4 ein guter Wärmeleiter, wie ihn Aluminium beispielsweise darstellt. Diese Behandlungsplatte 4 wird abgedeckt von einer weiteren festen Platte 7, die mittels mehrerer Schrauben 20 mit der Platte 7 zu einem Stapel 8 verbunden ist. Die Platte 7 kann aus demselben Material wie die Behandlungsplatte 4 bestehen, um Spannungen durch Temperaturunterschiede und durch unterschiedliche Ausdehnungskoeffizienten zu vermeiden. Bevorzugt kann die Platte 7 jedoch auch aus einem beispielsweise glasfaserverstärkten Kunststoff gefertigt sein, um der gesamten Platte größere mechanische Festigkeit zu geben und trotzdem den Wärmeübergang nach außen zu verringern. Die Körperwärme wird über die Kontaktfläche 2 und die Behandlungsplatte 4 in einen Wärmeträger geleitet. Der Wärmeträger durchströmt die Kanäle 5 in einem Zwangsumlauf. Zu diesem Zweck sind in geeigneter Weise in eine oder beide Platten 7, 4 Vertiefungen vorgesehen. Nach dem Zusammenfügen der beiden Platten 4, 7 zum Stapel 8 werden an den Grenzflächen 9, 10 Kanäle mit einem die Behandlungsplatte 4 gleichmäßig kühlender Strömungsweg gebildet. Um aus der umgebenden Raumumluft keine parasitäre Wärme mittels des Wärmeträgers 21 abführen zu müssen ist als dritte äußere Schicht eine den Stapel abdeckende Isolierschicht 11 vorgesehen, die beispielsweise aus einem verdichteten Hartpolyurethanschaum besteht und außen optisch ansprechend mit einer Folie oder einem Lack ausgerüstet ist.

Zur Unterstützung der Wirkung kann auch zusätzlich einer oder mehrere Ultraschallschwinger 18 und/oder Reizstromelektroden in der Behandlungsplatte vorgesehen sein. Die drei Schichten werden nach außen von einem Rahmen 14 umgeben, der auch als mechanischer Träger für die Halterung der Anschlussarmaturen zur Zufuhr des Wärmeträgers in die Kanäle 5 dient. In dem linken Anschnitt des Rahmens ist zusätzlich eine Öffnung 12 gezeigt, die zur Kontaktfläche 2 hin öffnet und unterdruckbeaufschlagt ist. Die Öffnung 12 mündet in einer Nut 13, die auf der unteren umlaufenden Fläche des Rahmens eingetieft ist. Wird der so beschriebene Applikator auf die Haut eines menschlichen Körpers gelegt und ausreichender Unterdruck in der Öffnung 12 erzeugt, so saugt sich der Applikator selbst an die Körperoberfläche an und stellt einen ausgezeichneten Wärmeübergang zu dem Körpergewebe her. Zusätzlich kann der Applikator auch von einer weiteren umlaufenden Dichtung umschlossen sein, um ein möglicherweise auftretenden Leckluftstrom zu verringern oder auszuschließen. Auf der rechten Seite der Figur 1 ist eine alternative Ausführung des Rahmens mit einer Wulstdichtung 24 dargestellt. Die erfindungsgemäße Adapterplatte kann das Körpergewebe nicht unter die Temperatur des Wärmeträgers herunter kühlen. Es reicht folglich aus, wenn die Temperatur des Wärmeträgers genau geregelt wird. Trotzdem kann es vorteilhaft sein, wenn auf der Oberfläche 3 der Behandlungsplatte 4 ein Temperatursensor 17, beispielsweise in Form eines Foliensensors angeordnet wird. Der Foliensensor behindert den Wärmeübergang nur unwesentlich und misst aber die tatsächlich aktuelle Gewebetemperatur an der Oberfläche 3. Das Signal dieses Sensors kann beispielsweise für die Protokollierung und die Regelung des Behandlungsverlaufs genutzt werden. Zur weiteren Komfortsteigerung bei der Behandlung dient ein VIies 16 das insbesondere auch aus hygienischen Gründen die Oberfläche 3 der Behandlungsplatte von der Körperoberfläche trennt und austauschbar ist. Wenn das Vlies luftdurchlässig ist, kann es zur Verbesserung der Hygiene die Dichtung mit überdeckend ausgebildet sein.

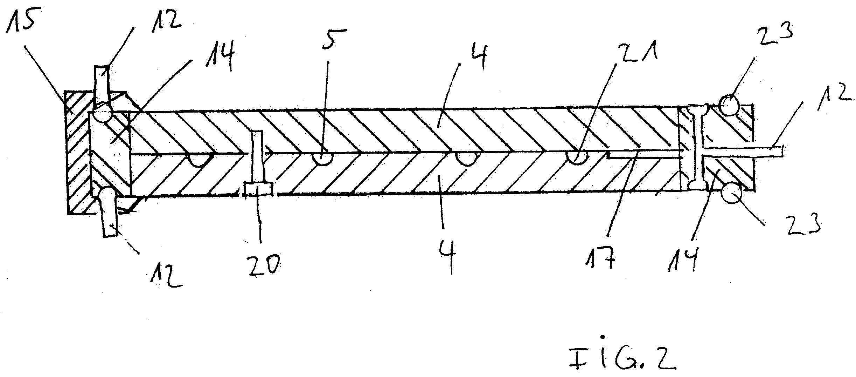

Figur 2 zeigt eine erfindungsgemäße Ausführungsform, bei der zwei Behandlungsplatten zu einem Stapel zusammengefügt sind. Ein solcher Applikator kommt z.B. bei der Behandlung der inneren Oberflächen von Oberschenkeln zum Einsatz. Dieser Applikator kann dann zwischen die Beine geklemmt werden. Die Isolierschicht 11 und die Platte 7 sind bei dieser Ausführung nicht notwendig. Im Übrigen ist die Funktionsweise dieses Applikators analog zu der wie im Zusammenhang mit Figur 1 beschrieben Funktionsweise.

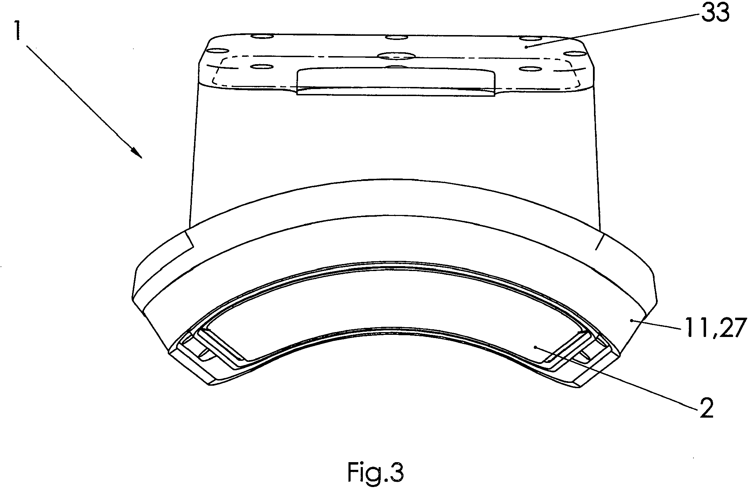

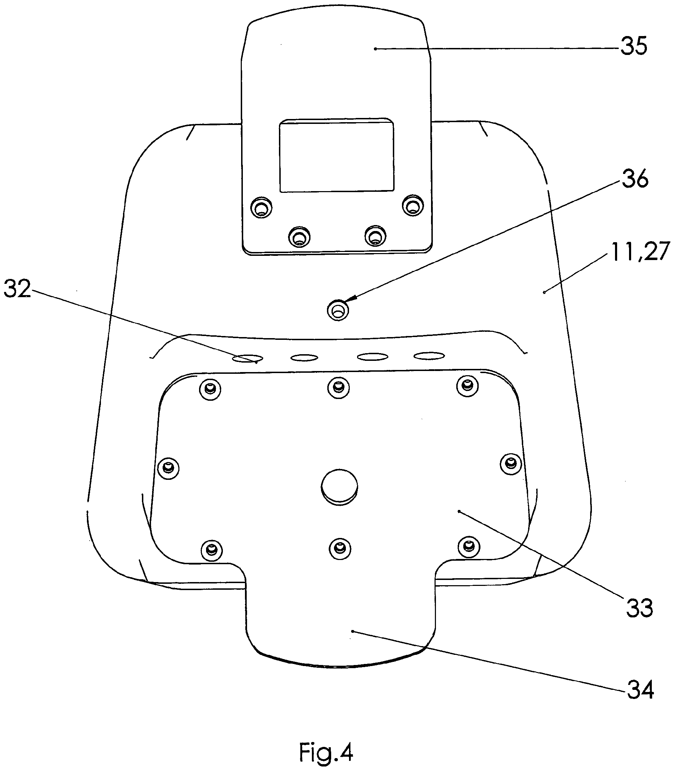

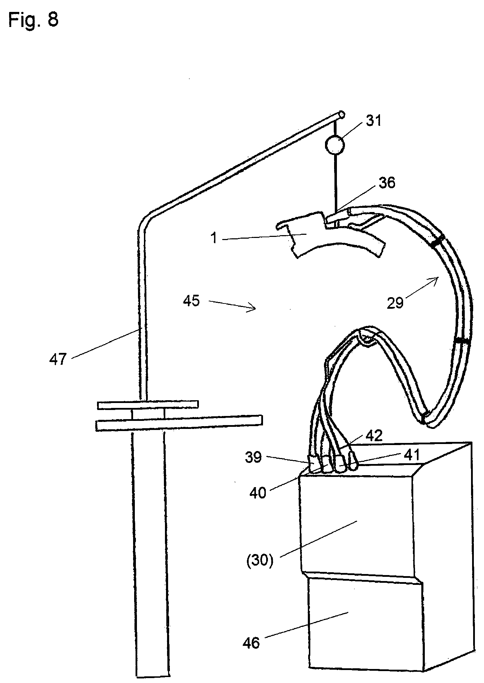

Die Figuren 3 bis 7 zeigen ein weiteres Ausführungsbeispiel des Applikators 1. Darin ist die Isolierschicht 11 zu einem Gehäuse 27 ausgeformt, in das Öffnungen 32 eingebracht sind für Anschlussarmaturen zur Zufuhr des Wärmeträgers und zum Verbinden etwaiger elektrischer Sensoren oder Ultraschallschwinger. Zwei der Öffnungen 32 dienen zur Montage der Anschlussarmaturen für Zulauf und Rücklauf des Wärmeträgers. Eine Öffnung steht für die Anschlussarmatur eines Vakuumschlauches zur Verfügung und eine vierte Öffnung dient zur Montage elektrischer Steckverbinder. Der Innenraum des Gehäuses 27 ist hinter einem aufgeschraubten Deckel 33 zugänglich, der gleichzeitig als Griff 34 ausgeformt ist. Mittels eines weiteren Griffs 35 lässt sich der Applikator trotz seines Gewichts mit zwei Händen leicht bewegen. Die Kontaktfläche 2 ist dabei quer zur Symmetrieachse konkav gebogen, um den Applikator 1 beispielsweise auf einen Oberschenkel aufzulegen. Ein zentral angeordneter Befestigungspunkt 36 dient zum Aufhängen des Applikators 1 an einem Balancier 31 (Figur 8).

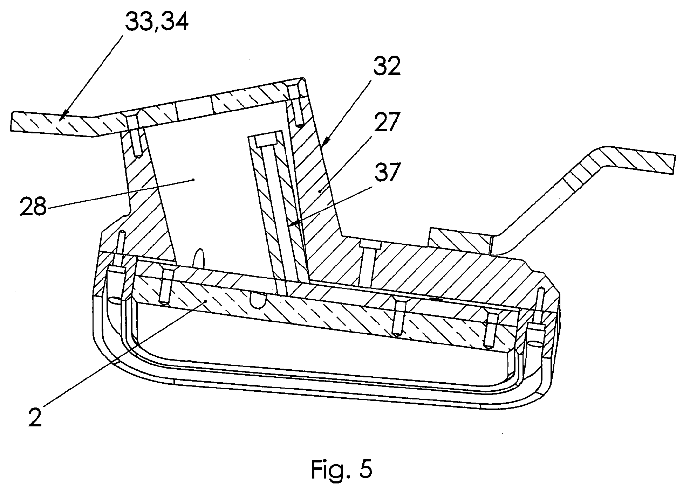

Figur 5 zeigt einen Vertikalschnitt entlang der Symmetrieachse des Gehäuses. Hinter den Öffnungen 32 ist eine Befestigungsplatte 37 angeordnet, mit korrespondierenden Montageöffnungen 38. Diese Montageöffnungen 38 sind hinter den Öffnungen 32 des Gehäuses angeordnet und dienen als Befestigungspunkte für den Wärmeträgerzulaufschlauch 39, den Rücklauf 40 des Wärmeträgers, den Vakuumschlauch 41 und das Kabel 42.

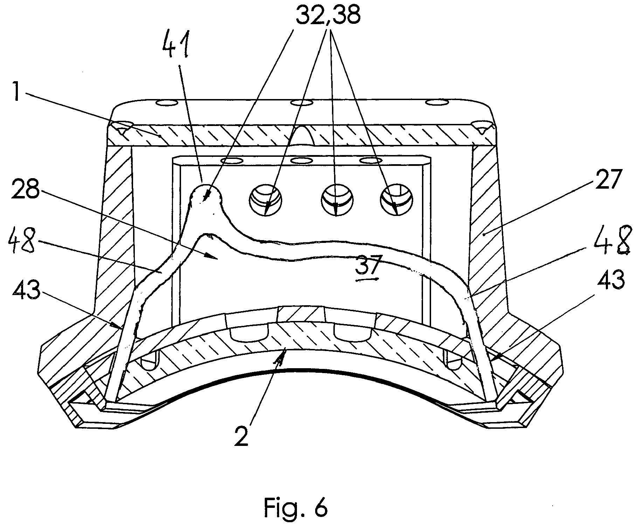

In Figur 6 ist eine Variante des Applikators 28 dargestellt, bei der der Innenraum 28 des Gehäuses 27 nicht mit Unterdruck beaufschlagt ist. Stattdessen wird der Unterdruck vom Vakuumschlauch 41 verteilt auf zwei Verbindungsschläuche 48, die den Unterdruck zu den Verbindungskanälen 43 führen.

Alternativ kann der Innenraum 28 des Gehäuses 27 mittels Vakuumschlauch 41 im Druck gegenüber der Atmosphäre abgesenkt sein. Dann entfallen die Verbindungsschläuche 48 zu den Verbindungskanälen 43, die den Innenraum 28 mit dem an die Kontaktfläche 2 angrenzenden von der umlaufenden Dichtung umschlossenen Raum verbinden. Dadurch saugt sich der Applikator an die darunter liegende Körperfläche an.

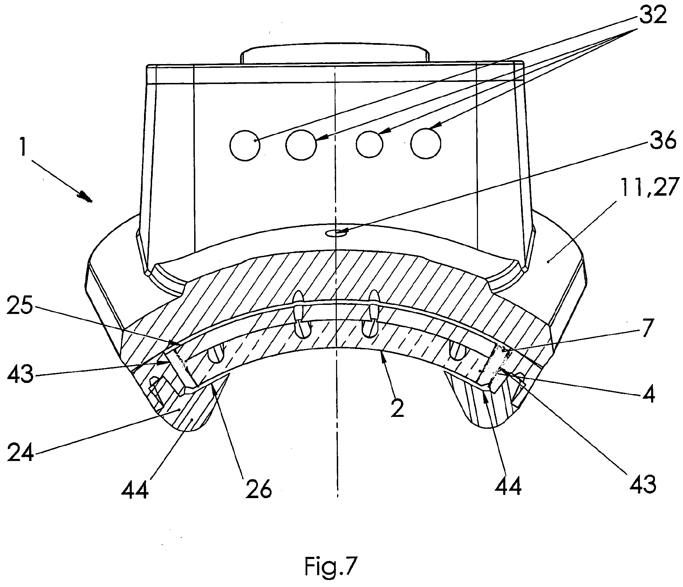

In dem Vertikalschnitt quer zur Symmetrieachse gemäß Figur 7 ist erkennbar, dass zwischen der Behandlungsplatte 4 mit weiterer Platte 7 und der Isolierschicht 11 ein Spalt 25 vorgesehen ist, der mit dem Innenraum 28 des Gehäuses 27 in Verbindung steht, und in dem deshalb ebenfalls Unterdruck herrscht. Von dort gelangt der Unterdruck über Verbindungskanäle 43 zur Kontaktfläche 2.

Die in Figur 7 gezeigte Wulstdichtung verläuft zur Kontaktfläche 2 hin in einem Übergangsbereich 26 keilförmig aus. Sie überdeckt in diesem Übergangsbereich 26 somit die Kontaktfläche 2 und verkleinert diese. Der Adapter 1 kann folglich auch an stärker gekrümmte Körperflächen angepasst werden. Dabei sorgen Kanäle 44 in der Dichtung, dass der Unterdruck die Kontaktfläche 2 erreicht.

Schließlich ist in Figur 8 das Gerät 45 gezeigt. Es besteht aus dem im Gehäuseblock 46 angeordneten Kältegerät 30 (verdeckt) zur Bereitstellung des Wärmeträgers, den verbindenden Versorgungsleitungen 29 zum Adapter 1 und dem Balancier 31, der den Adapter 1 praktisch schwerelos an der Halterung 47 griffbereit zur Verfügung hält.

Auf diese Weise steht ein Applikator 1 zur Behandlung von subkutanen fettreichen Zellen mit Kälte zur Verfügung, der hygienisch und besonders wirkungsvoll in Verbindung mit vorhandenen Systemen zur Kälteerzeugung benutzt werden kann.

**Bezugsziffernliste**

| | | | |
|---|---|---|---|
| 1 | Applikator | 25 | Spalt |
| 2 | Kontaktfläche | 26 | Übergangsbereich |
| 3 | Oberfläche | 27 | Gehäuse |
| 4 | Behandlungsplatte | 28 | Innenraum |
| 5 | Kanäle | 29 | Versorgungsleitung |
| 6 | Wärmeträger | 30 | Kältegerät |
| 7 | Platte | 31 | Balancier |
| 8 | Stapel | 32 | Öffnungen |
| 9 | Grenzfläche | 33 | Deckel |
| 10 | Grenzfläche | 34 | Griff |
| 11 | Isolierschicht | 35 | Griff |
| 12 | Öffnung | 36 | Befestigungspunkt |
| 13 | Nut | 37 | Befestigungsplatte |
| 14 | Rahmen | 38 | Montageöffnung |
| 15 | Dichtung | 39 | Zulauf |
| 16 | Vlies | 40 | Rücklauf |
| 17 | Temperatursensor | 41 | Vakuumschlauch |
| 18 | Ultraschallschwinger | 42 | Kabel |
| 19 | Stapel | 43 | Verbindungskanal |
| 20 | Schraube | 44 | Kanal |
| 21 | Wärmeträger | 45 | Gerät |
| 22 | Elektroden | 46 | Gehäuseblock |
| 23 | Dichtung | 47 | Halterung |
| 24 | Wulstdichtung | 48 | Verbindungsschlauch |

## Patentansprüche

1. Applikator zur Verwendung in Verbindung mit einem Gerät zur Behandlung von subkutanen fettreichen Zellen mit Kälte, wobei der Applikator (1) eine Kontaktfläche (2) aufweist, die als eine Oberfläche (3) einer festen Behandlungsplatte (4), vorzugsweise aus Aluminium oder einem anderen gut wärmeleitfähigen Material, geformt ist, **dadurch gekennzeichnet, dass** zwei Behandlungsplatten (4) zu einem Stapel (19) mit beidseitig angeordneten Kontaktflächen (2) verbunden sind.

2. Applikator nach Anspruch 1, **dadurch gekennzeichnet, dass** in der festen Behandlungsplatte (4) Kanäle (5) zum Leiten eines Wärmeträgers (6) vorgesehen sind.

3. Applikator nach Anspruch 1 oder 2, **dadurch gekennzeichnet, dass** die feste Behandlungsplatte (4) mit einer weiteren festen Platte (7) zu einem Stapel (8) geschichtet verbunden ist.

4. Applikator nach Anspruch 2, **dadurch gekennzeichnet, dass** die Kanäle (5) in einer Grenzfläche (9, 10) der beiden fest verbundenen Platten (4, 7) verlaufen.

5. Applikator nach Anspruch 3, **dadurch gekennzeichnet**, dasseine äußere den Stapel (8) abdeckende Isolierschicht (11) vorgesehen ist, wobei zwischen Isolierschicht (11) und weiterer fester Platte (7) ein Spalt (25) vorgesehen ist, der vorzugsweise vakuumbeaufschlagt ausgebildet ist.

6. Applikator nach Anspruch 3, **dadurch gekennzeichnet, dass** der Applikator (1) mindestens eine in oder um die Kontaktfläche (2) angeordnete unterdruckbeaufschlagte Öffnung (12) aufweist.

7. Applikator nach Anspruch 6, **dadurch gekennzeichnet, dass** die unterdruckbeaufschlagte Öffnung (12) in einer die Kontaktfläche (2) einrahmenden umlaufende Nut (13) mündend angeordnet ist.

8. Applikator nach Anspruch 7, **dadurch gekennzeichnet, dass** die Nut (13) in einem festen den Stapel (8) einfassenden Rahmen (14) angeordnet ist.

9. Applikator nach einem oder mehreren der vorhergehenden Ansprüche, **dadurch gekennzeichnet, dass** eine die Kontaktfläche (2) einrahmende umlaufende Dichtung (15) vorgesehen ist, die vorzugsweise leicht austauschbar ausgebildet ist.

10. Applikator nach Anspruch 9, **dadurch gekennzeichnet, dass** die Dichtung (15) in Richtung umschlossener Kontaktfläche (2) diese teilweise überdeckend und keilförmig auslaufend geformt ist und im Übergangsbereich (26) mindestens ein quer zum Verlauf der Dichtung (15) gerichteter Kanal (44) vorgesehen ist..

11. Applikator nach einem oder mehreren der vorhergehenden Ansprüche, **dadurch gekennzeichnet, dass** ein Temperatursensor (17) vorgesehen ist, der vorzugsweise auf der Kontaktfläche (2) angeordnet ist oder innerhalb der Platte (4) angebracht ist.

12. Applikators nach einem oder mehreren der vorhergehenden Ansprüche, **dadurch gekennzeichnet, dass** ein Ultraschallschwinger (18) in der Behandlungsplatte (4) vorgesehen ist und/oder die Kontaktfläche (2) in mindestens einer Richtung konkav gebogen ausgebildet ist.

13. Applikator nach einem oder mehreren der vorhergehenden Ansprüche, **dadurch gekennzeichnet, dass** er ein Gehäuse (27) aufweist, dessen Innenraum (28) vakuumbeaufschlagt ausgebildet ist und in das Versorgungsleitungen (29) vakuumdicht einmünden.

14. Gerät zur Behandlung von subkutanen fettreichen Zellen mit Kälte mit einem Wärmeträgerkreislauf (30) und einem von einem Wärmeträger (6) durchflossenen Applikator (1), gemäß mindestens einem der vorstehenden Ansprüche, zur Behandlung eines lebenden Körpers, **dadurch gekennzeichnet, dass** es aus einem aus einem Kältegerät (30), zur Bereitstellung des Wärmeträgers (6) mit einer bestimmten Temperatur und verbindenden Versorgungsleitungen (29) zum Applikator (1) sowie einem das Gewicht des Applikators kompensierenden Balancier (31) besteht.

## Claims

1. Applicator for use in connection with an apparatus for treatment of subcutaneous, fat-rich cells using cold, wherein the applicator (1) has a contact face (2) which is formed as a surface (3) of a rigid treatment plate (4), preferably from aluminium or another material with good heat conductivity, **characterized in that** two treatment plates (4) are connected to form a stack (19) with contact faces (2) arranged on both sides.

2. Applicator according to Claim 1, **characterized in that** channels (5) for conducting a heat transfer medium (6) are provided in the rigid treatment plate (4).

3. Applicator according to Claim 1 or 2, **characterized in that** the rigid treatment plate (4) is connected to a further rigid plate (7) to form a layered stack (8).

4. Applicator according to Claim 2, **characterized in that** the channels (5) run in a boundary surface (9, 10) of the two rigidly connected plates (4, 7).

5. Applicator according to Claim 3, **characterized in that** an outer insulation layer (11) is provided covering the stack (8), wherein a gap (25) is provided between insulation layer (11) and further rigid plate (7), to which gap (25) a vacuum is preferably applied.

6. Applicator according to Claim 3, **characterized in that** the applicator (1) has at least one opening (12) which is arranged in or around the contact face (2) and to which an underpressure is applied.

7. Applicator according to Claim 6, **characterized in that** the opening (12) to which an underpressure is applied is arranged leading into a circumferential groove (13) that frames the contact face (2).

8. Applicator according to Claim 7, **characterized in that** the groove (13) is arranged in a rigid frame (14) that encloses the stack (8).

9. Applicator according to one or more of the preceding claims, **characterized in that** a circumferential seal (15) is provided that frames the contact face (2), said seal (15) preferably being designed to be easily exchangeable.

10. Applicator according to Claim 9, **characterized in that** the seal (15), in the direction of the enclosed contact face (2), is shaped to partially cover the enclosed contact face (2) and to run out in a wedge shape, and at least one channel (44) directed transversely with respect to the course of the seal (15) is provided in the transition area (26).

11. Applicator according to one or more of the preceding claims, **characterized in that** a temperature sensor (17) is provided, which is preferably arranged on the contact face (2) or is mounted inside the plate (4).

12. Applicator according to one or more of the preceding claims, **characterized in that** an ultrasonic oscillator (18) is provided in the treatment plate (4) and/or the contact face (2) is designed curving in a concave shape in at least one direction.

13. Applicator according to one or more of the preceding claims, **characterized in that** it has a housing (27), of which the interior (28) is designed to be subjected to a vacuum and into which supply lines (29) run in a vacuum-tight manner.

14. Apparatus for treatment of subcutaneous, fat-rich cells using cold, with a heat transfer circuit (30) and an applicator (1) through which a heat transfer medium (6) flows, according to at least one of the preceding claims, for treatment of a living body, **characterized in that** it is composed of a refrigerating apparatus (30), for making available the heat transfer medium (6) with a defined temperature, and of connecting supply lines (29) to the applicator (1), and also of a balancer (31) that compensates the weight of the applicator.

## Revendications

1. Applicateur destiné à être utilisé en association avec un appareil pour le traitement par le froid de cellules graisseuses sous-cutanées, l'applicateur (1) présentant une surface de contact (2) qui est formée sous forme de surface (3) d'une plaque de traitement fixe (4), de préférence en aluminium ou en un autre matériau bon conducteur thermique, **caractérisé en ce que** deux plaques de traitement (4) sont assemblées pour former une pile (19) avec des surfaces de contact (2) disposées des deux côtés.

2. Applicateur selon la revendication 1, **caractérisé en ce que** des canaux (5) pour conduire un fluide caloporteur (6) sont prévus dans la plaque de traitement fixe (4).

3. Applicateur selon la revendication 1 ou 2, **caractérisé en ce que** la plaque de traitement fixe (4) est assemblée en couche avec une plaque fixe supplémentaire (7) pour former une pile (8).

4. Applicateur selon la revendication 2, **caractérisé en ce que** les canaux (5) s'étendent dans une surface limite (9, 10) des deux plaques (4, 7) assemblées fixement.

5. Applicateur selon la revendication 3, **caractérisé en ce qu'**il est prévu une couche d'isolation extérieure (11) recouvrant la pile (8), une fente (25) étant prévue entre la couche d'isolation (11) et la plaque fixe supplémentaire (7), laquelle fente est réalisée de préférence de manière à être sollicitée par le vide.

6. Applicateur selon la revendication 3, **caractérisé en ce que** l'applicateur (1) présente au moins une ouverture (12) sollicitée par une dépression et disposée dans ou autour de la surface de contact (2).

7. Applicateur selon la revendication 6, **caractérisé en ce que** l'ouverture (12) sollicitée par une dépression est disposée de manière à déboucher dans une rainure périphérique (13) encadrant la surface de contact (2).

8. Applicateur selon la revendication 7, **caractérisé en ce que** la rainure (13) est disposée dans un cadre fixe (14) enserrant la pile (8).

9. Applicateur selon l'une quelconque ou plusieurs des revendications précédentes, **caractérisé en ce qu'**il est prévu un joint d'étanchéité périphérique (15) encadrant la surface de contact (2), lequel est réalisé de préférence de manière à pouvoir être facilement remplacé.

10. Applicateur selon la revendication 9, **caractérisé en ce que** le joint d'étanchéité (15) est formé, dans la direction de la surface de contact entourée (2), de manière à recouvrir au moins en partie cette dernière et de manière à se terminer en forme de coin et au moins un canal (44) orienté transversalement par rapport à l'étendue du joint d'étanchéité (15) est prévu dans la zone de transition (26).

11. Applicateur selon l'une quelconque ou plusieurs des revendications précédentes, **caractérisé en ce qu'**il est prévu un capteur de température (17) qui est de préférence disposé sur la surface de contact (2) ou qui est monté à l'intérieur de la plaque (4).

12. Applicateur selon l'une quelconque ou plusieurs des revendications précédentes, **caractérisé en ce qu'**un oscillateur à ultrasons (18) est prévu dans la plaque de traitement (4) et/ou la surface de contact (2) est réalisée avec une courbure concave dans au moins une direction.

13. Applicateur selon l'une quelconque ou plusieurs des revendications précédentes, **caractérisé en ce qu'**il présente un boîtier (27) dont l'espace interne (28) est réalisé de manière sollicitée par le vide et dans lequel débouchent des conduites d'alimentation (29) étanches au vide.

14. Appareil pour le traitement par le froid de cellules graisseuses sous-cutanées, comprenant un circuit de fluide caloporteur (30) et un applicateur (1) parcouru par un fluide caloporteur (6) selon au moins l'une quelconque des revendications précédentes, pour le traitement d'un corps vivant, **caractérisé en ce qu'**il se compose d'un appareil frigorifique (30) pour fournir le fluide caloporteur (6) à une température déterminée et de conduites d'alimentation de connexion (29) conduisant à l'applicateur (1) ainsi que d'un moyen d'équilibrage (31) compensant le poids de l'applicateur.
